# EUROPEAN PATENT SPECIFICATION

(11) **EP 0 298 257 B1**
(45) Date of publication and mention of the grant of the patent: **18.11.1993**
(21) Application number: 88108931.2
(22) Date of filing: 03.06.1988
(51) Int. Cl.: A61M 5/00

(54) **Connection for catheters, perfusion units and flasks of liquid to be perfused**
Verbindungseinrichtung für Katheder, Perfusionseinheiten und Flaschen für Perfusionsflüssigkeit
Dispositif de connexion pour cathéters, ensembles de perfusion et bouteilles de liquide de perfusion

(30) Priority: 05.06.1987 ES 8701910 U
(43) Date of publication of application: 11.01.1989
(73) Proprietor: Segura Badia, Marcelo, Barcelona (ES); Institut Municipal d'Assistencia Sanitaria, Barcelona (ES)
(72) Inventor: SEGURA Badia, Marcelo, Barcelona (ES)
(74) Representative: Klunker . Schmitt-Nilson . Hirsch

(56) References cited:
- EP-A- 0 256 640
- AT-B- 153 934
- FR-A- 2 397 995
- US-A- 3 986 508

## Description

This invention relates to a connection for catheters, perfusion units and flasks of liquids to be perfused of the kind used when parenterally administering liquids.

German Patent No. 153934 discloses an air filtering cap aimed at filtering the air admitted into a recipient containing a medical fluid or a food product. The filtering body is of a porous nature and it is impregnated with an antiseptic product. The needle for the extraction or eventually filling-up of the product to be contained in the recipient will perforate the walls enclosing the filtering body to allow its passage into the mass of the product contained in the recipient. A shallow cavity is provided on the top of the chamber containing the filtering body in order to admit a quantity of liquid antiseptic product like alcohol or similar, to help in the disinfection of the needle.

EPA 0256640 published after the priority date of this application discloses a connection device for peritoneal dialysis showing a chamber to contain a sterilizing medium which is to be connected to an end part corresponding to the catheter line and to a terminal connected to the human body. The chamber contains a fluid to provide desinfection of the terminal which is connected to the human body at the beginning of the dialysis cycle and also for the desinfection of the terminal which is connected to the catheter line.

It is already known that the supply of serum for patients in emergency units or undergoing different medical treatments comprises the use of a recipient for the liquid in question with a flexible duct or perfusion unit, which also goes to the catheter applied to the patient's body. Experience has shown that where the catheter is connected to the perfusion unit, or this latter to the recipient holding the liquid to be perfused, cases of contamination by microorganisms of different types occur as is the case when the catheter is used for clinical controls or for diagnostic techniques.

The object of this invention is to eliminate the risk of contamination from microorganisms, preventing, in the case of their being present in the connection components between the recipients and the catheter, any infection and ensuring that the ducting between the flasks and the patient's body, including the catheter itself, remains free from infection by said microorganisms.

This object is achieved in accordance with the features of claim 1.

The antimicrobian product included in the chamber is capable of reducing the number of viable microorganisms so that if these were to reach the exterior of a connection component, the patient would remain free from contamination and from any possible resultant infection.

The component which reduces the quantity of microorganisms that may be present consists of a capsule holding the antimicrobian product capable of neutralizing the action of microorganisms. Said product could be for example iodine alcohol or similar. The capsule is a chamber, limited, if possible, by two end walls made of a flexible material, producing a watertight seal which allows for penetration with a needle, like for example rubber, gum, etc., and which can be traversed by a tubular needle, with or without internal mandrin and constitutes part of the connection component associated with the duct coming from the recipient containing the serum or biological liquid to be administered.

The device described herein may be applied in different ways which shall be expounded on in the description below and illustrated in the accompanying drawings which provide some of the different embodiments for the invention, as per the principles of the claims.

Figures 1 and 2 show the connection device being used with a rigid flask, glass for example, of the kind used for serotherapy and which require air to enter simultaneously in order to allow the liquid to flow. Figures 3 and 4 show use with a flexible flask of the kind used for parenteral nutrition, polyethylene for example, which as a result do not require air flow.

Figures 5, 6 and 7 show corresponding embodiments of the connection device.

Figures 8 and 9 offer another case in which the connection device is used on the junction between the end of the catheter and the duct coming from the flask of liquid to be perfused.

Figure 10 provides a section of an example of a needle from the connection component, with an internally fitted mandrin.

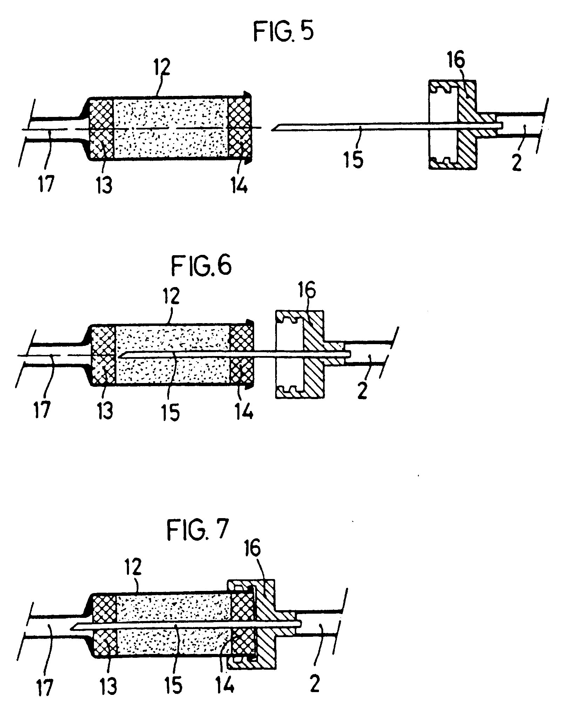

The device in question, comprises, for each of the abovementioned cases, a chamber -12- made from a cylindrical glass, rigid or semi-rigid polyethylene or other plastic body with, in a preferential version, rubber plugs -13- and -14- at each end. These may be penetrated by the tubular needle -15-, with or without mobile mandrin, which forms part of a connection component -16- associated to duct -2-, for the abovementioned cases. One end of the chamber -12- has one free end and the plug -14- is accessible so that it may be traversed by the needle -15-. The other end of the chamber -12- constitutes the continuation of the liquid duct -17-. The other plug -13- is set at a distance form the first so that it too may be traversed by the needle -15- as shown in figures 6 and 7.

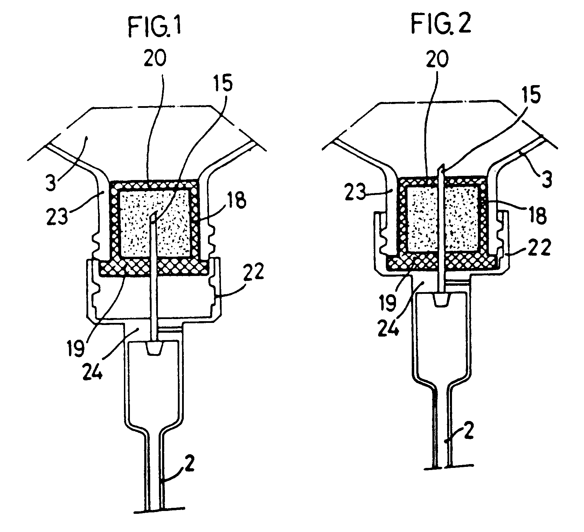

In the case of a rigid flask -3-, glass for example, as shown in figures 1 and 2, a cylindrical component -18- is applied to the lower opening of the recipient. One of the bases of this component, marked -19-, will be traversed by the needle -15-, with or without mandrin, which, after traversing said component, will perforate the other base -20- until it reaches the inside of the flask -3-, where it may collect the liquid to be perfused.

It is here that while the needle -15- is travelling longitudinally and for whatever time may be necessary, through the inside of the chamber that contains the antimicrobian product, in any form, liquid, gas, etc. (for example the aforementioned iodine alcohol), any contaminating microorganisms there may be in the connection components are reduced.

In the case shown in figures 1 and 2, the connection device comprises a screw plug -22- which screws on to the mouth -23- of the flask -3-, also threaded and, carries the tubular needle -15- attached to the partition -24-, to be extended into the duct -2- which wall link up with the catheter.

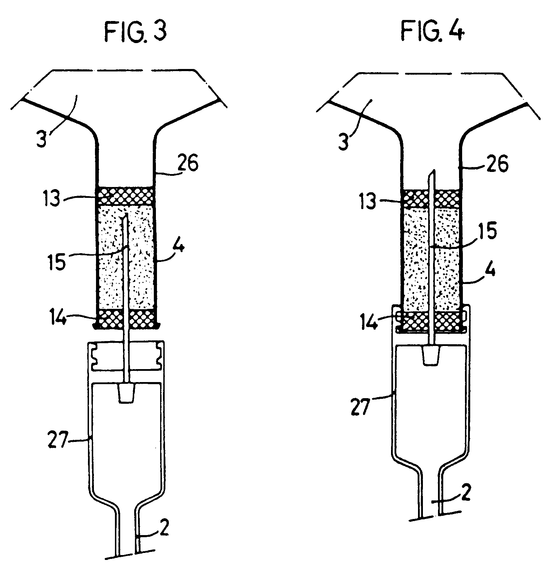

Figures 3 and 4 correspond to the case of a flask -3- made of a flexible material The device -4- is of the general type herein described, with a rigid body and flexible end plugs, traversed, one after the other, by the needle -15-, which wall finally perforate the rubber -13- in the flask mouth. The chamber -4- continues along the side -26- while at the other end it runs to the connection component -27- via the needle -15-.

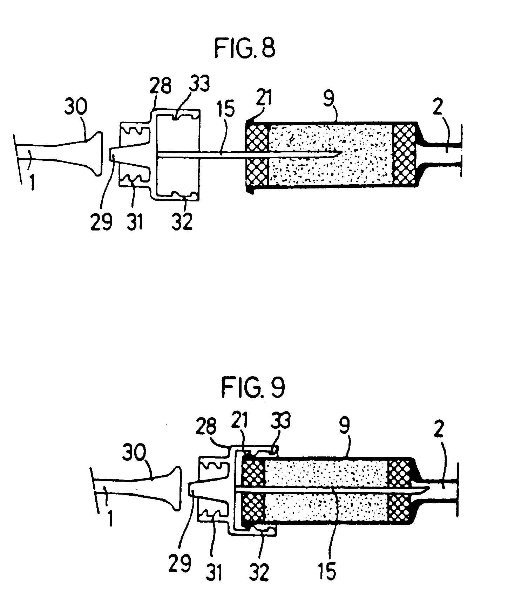

Figures 8 and 9 show the connection in use with an adaptor for catheters already in place. In these the linking method between the component -28-, connecting the end of the catheter -1- to the chamber -9-, as per the third case shown in figure 1, can be observed. In this component -28- a protuberance can be seen -29-. The wide mouth of the catheter -30- fits on to said protuberance while the cylindrical part -31- surrounds this mouth. The other cylindrical part -32-, of a larger diameter, has annular protuberances -33- which take the form of threads and grip on to the end of the chamber -9-, also provided with an annular protuberance -21-.

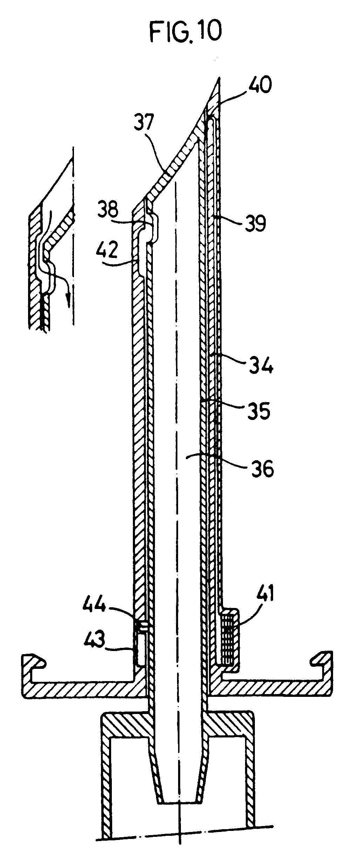

Figure 10 provides an example of a connection needle fitted with an internal mobile mandrin. The needle -34- contains a mandrin -35-, the central part of which is hollow -36-, with the end -37- closed and a lateral opening -38- near to said closed end -37-. It also has a duct -39- to allow air to pass to the end of the needle -40-. This external air is filtered before entry, through filter -41-. The inside of the needle has a cavity -42- at the top and another at the bottom -43- with a stop for the mandrin -44-, limiting the run of the mandrin's axial movements. When the mandrin is in the closed position, its end -37- blocks the needle outlet, so preventing any liquid escaping. When the mandrin is subjected to a downward axial movement, the liquid flows through the inside of the mandrin, entering through opening -38- and when the end of the mandrin -37- comes below needle cavity -42-, it flows through to the inside.

The results of the experiments carried out with the device described herein, are completely positive in that the presence of any microorganisms on the exterior part of the tubular needle -15- or the exterior parts of components -16-, -14- or 18, as per the different cases herein described, reduces the number of viable microorganisms, avoiding any possible effect they may have.

It is stated that everything not affecting, altering, changing or modifying the essence of the connection herein described shall be variable for the purposes of the present invention.

## Claims

1. Connection for catheters (1), perfusion units (2) and flasks (3) of liquids to be perfused of the kind used when parenterally administering liquids, which comprises a hermetically sealed chamber (4, 9, 12) and a connection component (16, 27, 28), the chamber containing an antimicrobian product and having an entry wall (14, 19) and an outlet wall (13, 20), the connection component carrying a connection needle (15) and having means (22, 32, 33) for connecting the catheter (1) or the perfusion unit (2) to the outside walls of the mouth (23) of a flask (3) carrying the chamber or to the outside walls of the chamber itself, the length of the connection needle (15) being greater than the length of the chamber including the entry wall and the outlet wall, whereby the connection needle in use will traverse the entry wall, the interior of the chamber and the outlet wall, providing a complete disinfection of the connection needle.

2. Connection for catheters (1) according to claim 1, in which the chamber (4, 9, 12) to contain the antimicrobian product is made of an integral enclosure of a semi-rigid plastic material which may be traversed by the extraction needle (15).

3. Connection for catheters (1) according to claim 1, in which the chamber (4, 9, 12) containing the antimicrobian product comprises a cylindrical body with said entry wall (14) and said outlet wall (13) at both ends of a material which can be penetrated by the needle (15).

4. Connection for catheters (1) according to claim 1, in which the needle (15) carrying the connection component is integral with a cap (22) which has retention means engageable with the outside surface of the chamber or with the external surface of the enclosure (3) containing the same.

5. Connection for catheters (1), according to any of the previous claims, in which the connection needle (15) may optionally have an interior mandrin (35) which allows the outlet through which the liquid to be perfused flows, to be opened or closed.

## Patentansprüche

1. Verbindungsvorrichtung für Katheter (1), Perfusionseinheiten (2) und Flaschen für Perfusionsflüssigkeiten, wie sie beim parenteralen Verabreichen von Flüssigkeiten verwendet werden, umfassend eine hermetisch abgedichtete Kammer (4, 9, 12) und ein Verbindungsbauteil (16, 27, 28), wobei die Kammer ein Bakterienwachstum verhinderndes Produkt enthält und eine Eintrittswand (14, 19) sowie eine Auslaßwand (13, 20) aufweist, das Verbindungsbauteil eine Verbindungsnadel (15) trägt und Mittel (22, 32, 33) aufweist zum Verbinden des Katheters (1) oder der Perfusionseinheit (2) mit den Außenwänden der Mündung (23) einer die Kammer tragenden Flasche (3) oder den Außenwänden der Kammer selbst, wobei die Länge der Verbindungsnadel (15) größer ist als die Länge der Kammer einschließlich der Eintrittswand und der Auslaßwand, wodurch die Verbindungsnadel im Gebrauch die Eintrittswand, das Innere der Kammer und die Auslaßwand durchsetzt und eine vollständige Desinfektion der Verbindungsnadel schafft.

2. Verbindungsvorrichtung für Katheter (1) nach Anspruch 1, bei der die Kammer (4, 9, 12) für das Bakterienwachstum verhindernde Produkt aus einer einstückigen Umschließung aus halbstarrem Kunststoffmaterial besteht, die von der Extraktionsnadel (15) durchsetzt wird.

3. Verbindungsvorrichtung für Katheter (1) nach Anspruch 1, bei der die Kammer (4, 9, 12), welche das Bakterienwachstum verhindernde Produkt enthält, einen zylindrischen Körper aufweist, an dessen beiden Enden sich die Eintrittswand (14) und die Auslaßwand (13) aus einem Material befinden, welches von der Nadel (15) durchdringbar ist.

4. Verbindungsvorrichtung für Katheter (1) nach Anspruch 1, bei der die das Verbindungsbauteil tragende Nadel (15) einstückig mit einer Kappe (22) ausgebildet ist, die Haltemittel besitzt, die mit der Außenfläche der Kammer oder mit der Außenfläche der diese enthaltenden Umhüllung (3) in Eingriff bringbar sind.

5. Verbindungsvorrichtung für Katheter (1) nach irgendeinem der vorhergehenden Ansprüche, bei der die Verbindungsnadel (15) wahlweise einen Innendorn (35) aufweisen kann, der ein Öffnen oder ein Schließen des Auslaß ermöglicht, durch den hindurch die Perfusionsflüssigkeit fließt.

## Revendications

1. Dispositif de branchement pour cathéters (1), unités de transfusion (2) et fioles ou poches (3) de liquides à transfuser du type utilisé pour l'administration parentérale de liquides qui comprend une chambre rendue hermétiquement étanche (4, 9, 12) et un composant de branchement (16, 27, 28), la chambre contenant un produit antimicrobien et possédant une paroi d'entrée (14, 19) et une paroi de sortie (13, 20), le composant de branchement supportant une aiguille de branchement (15) et comportant des moyens (22, 32, 33) pour le branchement du cathéter (1) ou de l'unité de transfusion (2) sur les parois extérieures de l'embouchure (23) d'une fiole (3) supportant la chambre ou sur les parois extérieures de la chambre elle-même, la longueur de l'aiguille de branchement (15) étant supérieure à la longueur de la chambre comprenant la paroi d'entrée et la paroi de sortie, l'aiguille de branchement utilisée traversant la paroi d'entrée, l'intérieur de la chambre et la paroi extérieure, assurant une désinfection totale de l'aiguille de branchement.

2. Dispositif de branchement pour cathéters (1) selon la revendication 1, dans lequel la chambre (4, 9, 12) devant contenir le produit antimicrobien est réalisé à partir d'une enceinte solidaire en plastique semi-rigide qui peut être traversée par l'aiguille d'extraction (15).

3. Dispositif de branchement pour cathéters (1) selon la revendication 1, dans lequel la chambre (4, 9, 12) contenant le produit antimicrobien comprend un corps cylindrique avec la paroi d'entrée (14) et la paroi de sortie (13) aux deux extrémités d'un matériau pouvant être traversé par l'aiguille (15).

4. Dispositif de branchement pour cathéters (1) selon la revendication 1, dans lequel l'aiguille (15) portant le composant de branchement est solidaire d'un capuchon (22) qui possède des moyens de retenue pouvant coopérer avec la surface extérieure de la chambre ou avec la surface externe de l'enceinte (3) contenant celle-ci.

5. Dispositif de branchement pour cathéters (1) selon l'une quelconque des revendications précédentes, dans lequel l'aiguille de branchement (15) peut facultativement comporter un mandrin intérieur (35) qui permet l'ouverture ou la fermeture de l'orifice de sortie à travers lequel s'écoule le liquide à transfuser.
